# EUROPEAN PATENT APPLICATION

(11) **EP 4 166 115 A1**
(43) Date of publication of application: **19.04.2023**
(21) Application number: 22201630.5
(22) Date of filing: 14.10.2022
(51) Int. Cl.: A61F 2/40

(54) **MOBILE BEARING ANATOMIC GLENOID**

(30) Priority: 15.10.2021 US 202163262567 P; 13.10.2022 US 202218046297
(71) Applicant: Howmedica Osteonics Corp., Mahwah, NJ 07430 (US)
(72) Inventor: WOLFE, Alexander Paul, Fort Wayne, 46804 (US); PENNINGER, Charles L., Warsaw, 46580 (US); COURTNEY, Robert, Jr., Pierceton, 46562 (US)
(74) Representative: Zimmermann & Partner Patentanwälte mbB

(57) **Abstract**

Provided is a glenoid implant system (100) that includes a tray component (120) that includes a bone-facing surface (122) and a bearing-facing surface (123) on opposite side of the bone-facing surface; and a bearing component (130) that includes an articulating surface (132) that is generally concave and a back side (133) on opposite side of the articulating surface that is generally convex, wherein the articulating surface is configured for engaging a convex humeral head and the back side is configured for engaging the bearing-facing surface of the tray component thus enabling the bearing component to slide about on the tray component while the convex humeral head articulates against the articulating surface.

## Description

### CROSS-REFERENCE TO RELATED APPLICATIONS

This application claims priority under 35 U.S.C. § 119(e) to United States Provisional Application No. 63/262,567, filed on October 15, 2021, the entire contents of which are incorporated herein by reference.

### FIELD OF DISCLOSURE

The present disclosure generally relates to glenoid implants for shoulder prosthesis.

### BACKGROUND

A shoulder prosthesis includes a glenoid implant intended to replace the glenoid cavity of the scapula and/or a humeral implant intended to replace the humeral head. The glenoid implant generally includes an articular body intended to articulate with the humeral head, and a fixation means to stabilize the articular body with respect to the scapula.

Currently, clinical literature shows a high rate of radiolucency around glenoid cemented, non-cemented, and hybrid components in long-term use of those glenoid implants. One issue is the potential for rocking of the implant when shoulder joint is subjected to daily living activities. Currently, cemented implants provide good short- and mid-term fixation, but loosen over time. The current hybrid cemented-press fit implants show similar performance. Press fit implants historically show high loosening at mid- to long-term time points.

One of the challenges with press-fit glenoid implants is that it is difficult to reliably secure the implant to the bone, which is why hybrid cemented-press fit implants have increased in popularity. Adding a modular metal tray with screws is a solution that is used in many other joints, however in the shoulder there is often insufficient space for a modular tray. In addition, a modular connection between the metal tray and the bearing component may present a risk of unintended modular component separation, compared to a single piece implant.

Thus, improved glenoid implant design that offers enhanced and durable primary fixation to the bone is desired.

### SUMMARY

Provided is a glenoid implant system comprising a tray component that comprises a bone-facing surface and a bearing-facing surface on opposite side of the bone-facing surface; and
a bearing component that comprises an articulating surface that is generally concave and a back side on opposite side of the articulating surface that is generally convex, wherein the articulating surface is configured for engaging a convex humeral head and the back side is configured for engaging the bearing-facing surface of the tray component enabling the bearing component to slide about on the tray component while the convex humeral head articulates against the articulating surface.

In some embodiments, the glenoid implant system comprises a motion limiting feature that limits the range of motion of the bearing component sliding about the bearing-facing surface of the tray component.

In some embodiments, the motion limiting feature can comprise the bearing-facing surface being defined by more than one radii of curvature wherein the bearing-facing surface comprises: a central region that is a spherical surface having a curvature defined by the first radius of curvature; and a peripheral portion that is a spherical surface having a curvature defined by a second radius of curvature that is smaller than the first radius of curvature.

In some embodiments, the motion limiting feature can comprise a continuous ridge that extends along the bearing-facing surface's perimeter and is raised from the bearing-facing surface away from the bone-facing surface.

In some embodiments, the motion limiting feature can comprise two or more ridge segments that extend along the bearing-facing surface's perimeter and are raised from the bearing-facing surface away from the bone-facing surface. The motion limiting feature wherein the motion limiting feature comprises a boss provided on the back side of the articulating component and a pocket provided on the bearing-facing surface of the tray component, wherein the pocket has an opening that is larger than the boss.

In some embodiments, the motion limiting feature comprises a pocket provided on the back side of the articulating component and a boss provided on the bearing-facing surface of the tray component, wherein the pocket has an opening that is larger than the boss.

### BRIEF DESCRIPTION OF THE DRAWINGS

The various embodiments of the inventive hydrogel implant of the present disclosure will be described in more detail in conjunction with the following drawing figures. The structures in the drawing figures are illustrated schematically and are not necessarily intended to show actual dimensions or relative scale.

The glenoid implant system of Claim 14, comprising a motion limiting feature that limits the range of motion of the bearing component sliding about the bearing-facing surface of the tray component.
FIG. 1A is an illustration showing an isometric view of an example of the glenoid bearing implant system viewed from the articulating surface side.
FIG. 1B is an illustration of another view of the glenoid bearing implant system of the present disclosure.
FIGS. 2A-2D are illustrations showing various views of the mobile bearing component of the glenoid bearing implant system of the present disclosure.
FIG. 3 is an cross-sectional illustration of the glenoid bearing implant system shown in FIG. 1A.
FIG. 4 is an illustration showing the bond-contacting surface side of the glenoid bearing implant system shown in FIG. 1A.
FIG. 5 is an illustration showing another embodiment of a tray component of the glenoid bearing implant system of the present disclosure.
FIG. 6 is an illustration sowing a glenoid bearing implant system that includes the tray component shown in FIG. 5 according to another embodiment.
FIG. 7 is an illustration showing an isometric view of another example of the glenoid bearing implant system.
FIGS. 8A-8C are illustrations showing cross-sectional views of the glenoid bearing implant system of FIG. 7.
FIG. 9 is an illustration showing an isometric view of another example of the glenoid bearing implant system.
FIGS. 10A-10C are illustrations showing cross-sectional views of the glenoid bearing implant system of FIG. 9.

### DETAILED DESCRIPTION

This description of the exemplary embodiments is intended to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

Provided herein are various improved glenoid bearing implants that have articulation surface that is configured to engage with an anatomical humeral head or a humeral component of a shoulder replacement implant system. Therefore, references to "a humeral head" as used herein should be construed to include both an anatomical humeral head as well as implant humeral head. Particularly, the improved glenoid bearing implant system disclosed herein comprises a tray component that is configured to be secured to a surface on a glenoid of a patient that is appropriately prepared and a bearing component that is not afixed to the tray component but rather configured to slidingly engage the tray component so that the bearing component is positioned between the tray component and the patient's humeral head when the glenoid implant system is implanted in the patient's shoulder. The bearing component in this position is relatively unconstrained while in engagement with the humeral head and the tray component and can slide about against the tray component through the patient's articulation of the shoulder joint. As will be described below, the implant components can be configured to limit the range of motion for the bearing component.

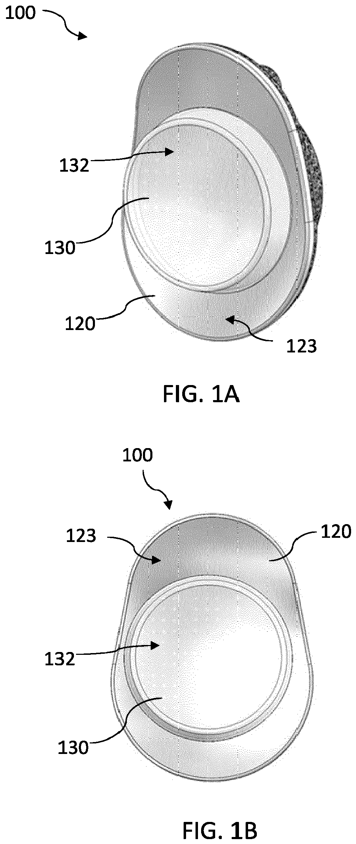

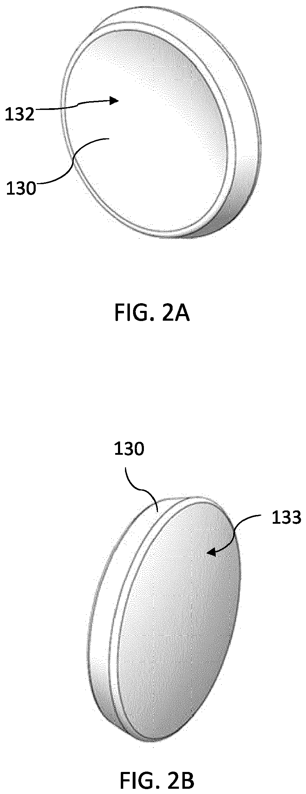

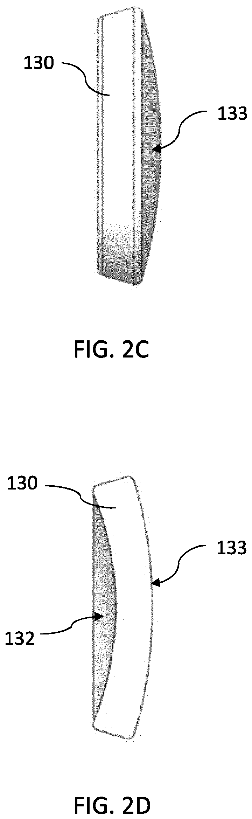

Referring to FIGS. 1A-4, an embodiment of an improved glenoid implant system **100** for implantation in a glenoid according to the present disclosure is provided. The glenoid implant system **100** comprises the tray component **120** and the bearing component **130.** The tray component **120** includes a bone-facing surface **122** and a bearing-facing surface **123** on the opposite side of the bone-facing surface **122.** The bearing component **130** includes an articulating surface **132** and a back side **133** on the opposite side of the articulating surface **132.**

The backside **133** of the bearing component **130** and the bearing-facing surface **123** of the tray component **120** have surfaces that are configured to engage each other in a sliding manner. The surface of the backside **133** has a generally convex contour and the bearing-facing surface **123** has a corresponding generally concave surface contour.

The articulating surface **132** of the bearing component **130** is generally concave. The articulating surface **132** is configured for engaging a convex humeral head and the back side **133** is generally convex and is configured for engaging the bearing-facing surface.

In some embodiments, the back side **133** of the bearing component **130** can be a spherical surface having a curvature defined by a first radius of curvature. In some embodiments, the bearing-facing surface **123** of the tray component can be a spherical surface having a curvature defined by the first radius of curvature.

In some embodiments, the glenoid implant system **100** can be provided with a motion limiting feature that limits the range of motion of the bearing component **130** while the bearing component **130** slides about on the tray component **120** while a convex humeral head articulates against the articulating surface after the glenoid implant system **100** has been implanted in a patient.

In some embodiments, the bearing-facing surface **123** has a complex contour defined by more than one radii of curvature. For example, the bearing-facing surface **123** in the central region can be defined by the first radius of curvature and the bearing-facing surface **123** along the periphery surrounding the central region can be defined by a second radius of curvature that is smaller than the first radius of curvature. This results in the central region that is flatter than the periphery and the surface of the periphery form a steeper curved surface surrounding the central region. The central region of the bearing-facing surface **123** is where the bearing component **130** is generally engaged and slides about relative to the tray component **120.** Thus, as the bearing component **130** slides toward the periphery of the tray component **120** the bearing-facing surface **123** presents a steeper curvature surface and hinders or prevents the bearing component **130** from sliding further. Therefore, the smaller radius of curvature along the periphery of the bearing-facing surface **123** functions to limit the range of motion for the bearing component **130** to keep the bearing component **130** engaged to the tray component **120** during a patient's shoulder joint articulation after the glenoid implant system **100** is implanted in the patient's shoulder.

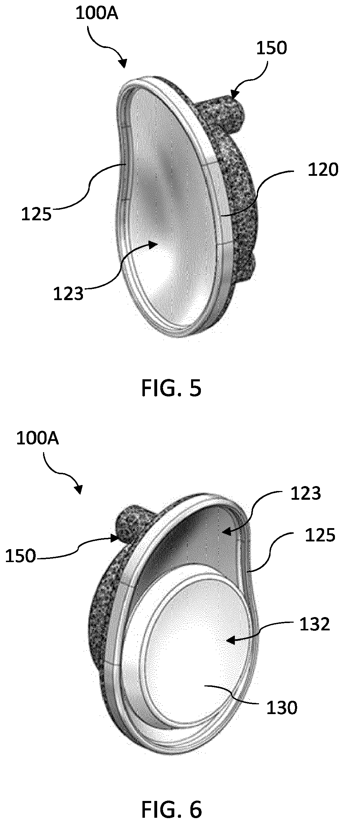

Referring to FIGS. 5-6, another example of a motion limiting feature is disclosed. In these embodiments, the bearing-facing surface **123** of the tray component **120** comprises a ridge **125** that extends along the bearing-facing surface's perimeter and is raised from the bearing-facing surface **123** in the direction leading away from the bone-facing surface **123** as shown. The ridge **125** functions as a retaining wall or a bumper that keeps the bearing component **130** engaging the bearing-facing surface **123** and keep the bearing component **130** from sliding off the surface of the tray component **120** when the glenoid implant system **100** is installed in a patient.

In some embodiments, the ridge **125** can extend completely around the perimeter of the bearing-facing surface **123** without any breaks as shown in the example shown in FIGS. 5 and 6. In some embodiments, the ridge **125** can be discontinuous. In other words, the ridge **125** can include one or more breaks. If there are more than one break, the ridge **125** will comprise two or more ridge segments that extend along the perimeter of the bearing-facing surface **123.**

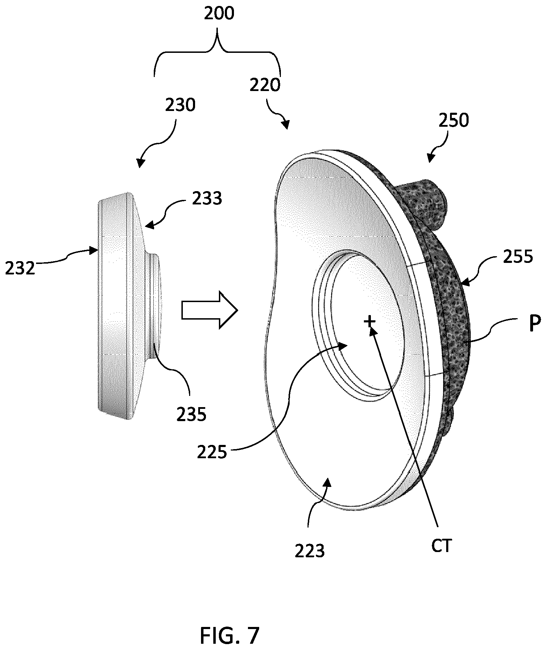

Referring to FIG. 7, a glenoid implant system **200** according to another embodiment that incorporates another example of a motion limiting feature is disclosed. The glenoid implant system **200** comprises a bearing component **230** and a tray component **220.** The tray component **220** includes a bone-facing surface **222** and a bearing-facing surface **223** on the opposite side of the bone-facing surface **222.** The bearing component **230** includes an articulating surface **232** and a back side **233** on the opposite side of the articulating surface **232.**

The backside **233** of the bearing component **230** and the bearing-facing surface **223** of the tray component **220** have surfaces that are configured to engage each other in a sliding manner but with a predefined limited range of motion for the bearing component **230** so that the outer edges of the bearing component **230** does not travel beyond the perimeter of the tray component **220.** The surface of the backside **233** has a generally convex contour and the bearing-facing surface **223** has a complementary generally concave surface contour but each is provided with a motion limiting feature that cooperate with one another.

The motion limiting feature on the backside **233** of the bearing component is a boss **235.** The motion limiting feature on the bearing-facing surface **223** of the tray component **220** is a corresponding pocket **225.** The pocket **225** has an opening that is larger than the boss **235** so that when the backside **233** of the bearing component **230** engages the bearing-facing surface **223** of the tray component, the boss **235** is completely received in the pocket **225** and allow the bearing component **230** to slide about against the bearing-facing surface **223.**

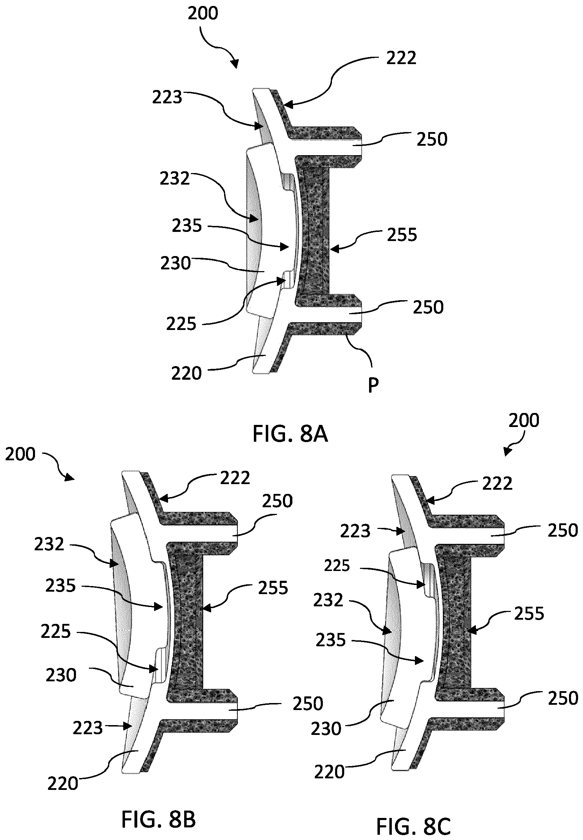

FIGS. 8A-8C show cross-sectional views of the bearing component **230** engaged with the tray component **220** where the boss **235** is received within the pocket **225.** In FIG. 8A, the bearing component **230** is situated so that the boss **235** is inside the pocket **225** without contacting the edges of the pocket **225.** In FIG. 8B, the bearing component **230** is slid upward in the maximum amount possible until the boss **235** is in contact with the edge of the pocket **225.** In this context, the direction "up" in "upward" refers to the direct toward the top of the page containing FIG. 8B. In FIG. 8C, the bearing component **230** is slid downward in the maximum amount possible until the boss **235** is in contact with the edge of the pocket **225.** In this context, the direction "down" in "downward" refers to the direction toward the bottom of the page containing FIG. 8C.

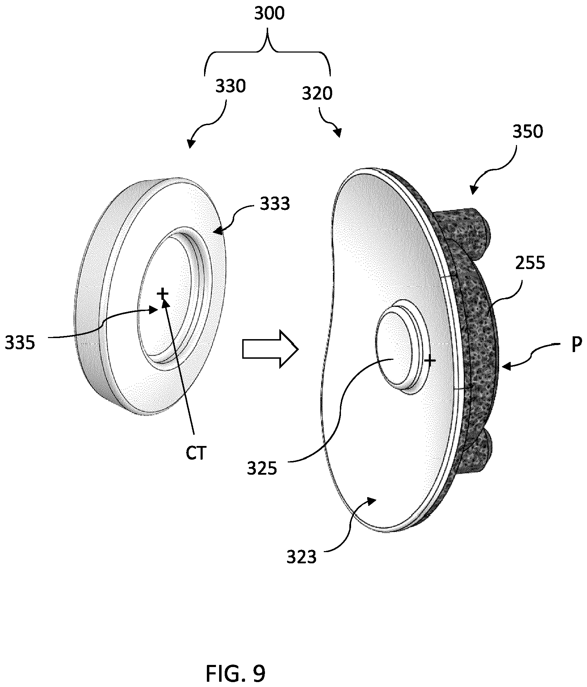

Referring to FIG. 9, a glenoid implant system **300** according to another embodiment that incorporates another example of a motion limiting feature is disclosed. The glenoid implant system **300** comprises a bearing component **330** and a tray component **320.** The tray component **320** includes a bone-facing surface **322** and a bearing-facing surface **323** on the opposite side of the bone-facing surface **322.** The bearing component **330** includes an articulating surface **332** and a back side **333** on the opposite side of the articulating surface **332.**

The backside **333** of the bearing component **330** and the bearing-facing surface **323** of the tray component **320** have surfaces that are configured to engage each other in a sliding manner but with a predefined limited range of motion for the bearing component **330** so that the outer edges of the bearing component **330** does not travel beyond the perimeter of the tray component **320.** The surface of the backside **333** has a generally convex contour and the bearing-facing surface **323** has a complementary generally concave surface contour but each is provided with a motion limiting feature that cooperate with one another.

In the glenoid implant system **300,** the motion limiting features on the bearing component **330** and the tray component **320** are opposite of those in the glenoid implant system **200.** The motion limiting feature on the backside **333** of the bearing component is a pocket **335.** The motion limiting feature on the bearing-facing surface **323** of the tray component **320** is a corresponding boss **325.** The pocket **335** has an opening that is larger than the boss **325** so that when the backside **333** of the bearing component **330** engages the bearing-facing surface **323** of the tray component, the boss **325** is completely received in the pocket **335** and allow the bearing component **330** to slide about against the bearing-facing surface **323.**

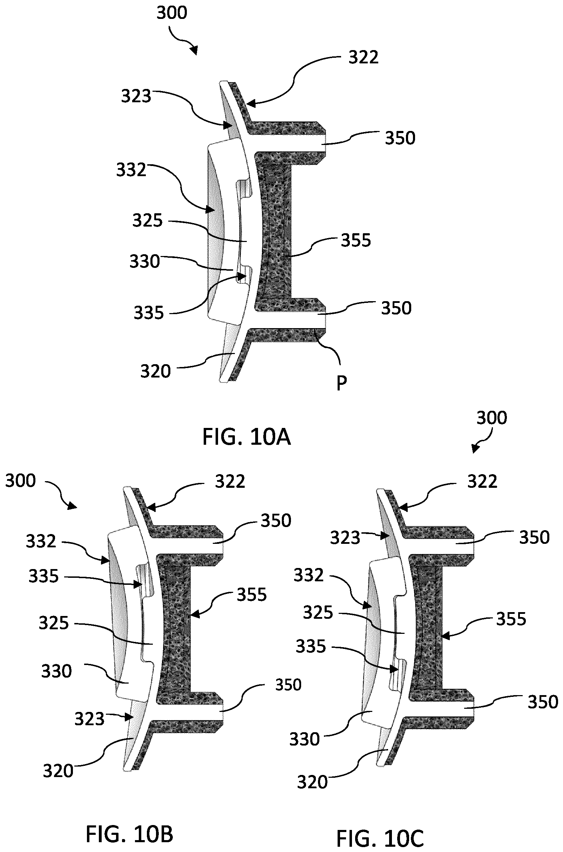

FIGS. 10A-10C show cross-sectional views of the bearing component **330** engaged with the tray component **320** where the boss **325** is received within the pocket **335.** In FIG. 10A, the bearing component **330** is situated so that the boss **325** is inside the pocket **335** without contacting the edges of the pocket **335.** In FIG. 10B, the bearing component **330** is slid upward in the maximum amount possible until the boss **325** is in contact with the edge of the pocket **335.** In this context, the direction "up" in "upward" refers to the direct toward the top of the page containing FIG. 10B. In FIG. 10C, the bearing component **330** is slid downward in the maximum amount possible until the boss **325** is in contact with the edge of the pocket **335.** In this context, the direction "down" in "downward" refers to the direction toward the bottom of the page containing FIG. 10C.

In the examples shown in FIGS. 7 and 9, the boss **235, 325** and the pocket **225, 335** are circular in shape. This would provide the boss **235, 325** with a maximum range of motion within the respective pocket **225, 335** that is defined by the circular shape of the pocket **225, 335.** This results in the maximum range of motion for the bearing component **230, 330** that is also circular shaped so that the bearing component **230, 330** is able to be move about equally in 360° of direction about the center **CT** of the pocket **225, 335.** In other embodiments, the shape of the pocket **225, 335** and/or the boss **235, 325** can be customized in non-circular shape to define a range of motion for the bearing component **230, 330** that is something other than a circular shape. For example, the pocket **225, 335** can be configured in an oval shape.

In some embodiments, the bearing components **130, 230, 330** can be formed of a high-modulus polymer. For example, the bearing component can be formed of high-modulus polyethylene (HMPE), ultra-high-molecular-weight polyethylene (UHMWPE), or any of the known polyethylene.

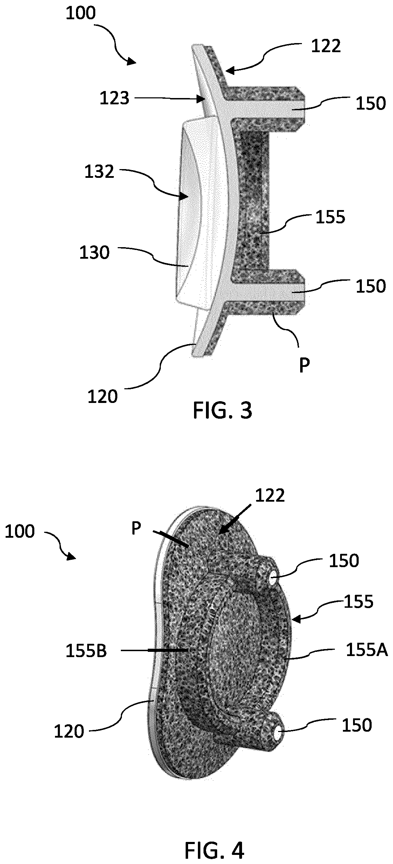

Referring to FIGS. 3, 4, 8A, and 10A in some embodiments, the bone-facing surface **122, 222, 322** of the tray component **120, 220, 320** can be treated or otherwise configured to enhance adherence to the bone. In some embodiments, the bone-facing surface **122, 222, 322** comprises at least a portion that has a layer of a porous material **P** that can promote bone in-growth. Some examples of such porous material are porous metallic materials such as Tritanium^{®} and ADAPTIS^{™} by Stryker Corporation. Another option can be plasma sprayed porous metallic materials. In some embodiments, the bone-facing surface **122, 222, 322** comprises at least a portion that is a roughened surface (formed by grit blasting or mechanically engraving) and bone cement can be used to bond the tray component **120, 220, 320** to the bone.

In some embodiments, the bone-facing surface **122, 222, 322** further comprises one or more peripheral fixation features **150, 155, 250, 255, 350, 355.** The peripheral fixation features extend from the bone-facing surface **122, 222, 322** in a direction that is away from the bearing-facing surface as shown in FIGS. 3, 4, 8A, and 10A.

In some embodiments, the one or more peripheral fixation features can be posts **150, 250, 350.** In other embodiments, the one or more peripheral fixation features can be provided in other structural forms such as rings, keels, screws, center post, etc. In some embodiments, the bone-facing surface **122, 222, 322** can be configured with one or more posts as at least part of the one or more peripheral fixation features.

In some embodiments, the one or more peripheral fixation features is an annular ring **155, 255, 355.** The annular ring **155, 255, 355** can be configured into two or more segments. For example, in the example shown in FIGS. 3 and 4, the annular ring **155** structure is interrupted by two posts **150** and defines the annular ring **155** into two segments **155A** and **155B.** In some embodiments, the one or more posts **150** can extend from the annular ring **155.** The annular ring **255** of the tray component **220,** and the annular ring **355** of the tray component 320 are configured in a similar manner.

In some embodiments, the one or more peripheral fixation features **150, 155, 250, 255, 350, 355** also can comprise a layer of a porous material so that at least some portion of the bone-facing surface **122, 222, 322** that comprises a layer **P** of a porous material can include the surfaces of the one or more peripheral fixation features **150, 155, 250, 255, 350, 355** that comprise a layer of porous material.

In some embodiments, the bearing component **130, 230, 330** can be formed of a high-modulus polymer. For example, the bearing component can be formed of high-modulus polyethylene (HMPE), ultra-high-molecular-weight polyethylene (UHMWPE), or any of the known polyethylene.

In some embodiments, the tray component **120, 220, 320** can be formed of a biocompatible metal alloys well known in the art. The bearing-facing surface **123, 223, 323** of the tray component can be a polished and hardened surface. The tray component **120, 220, 320** can be formed of a titanium alloy such as Ti-6Al-4V (also known as TC4 or Ti64). In some embodiments, the tray component **120, 220, 320** can be formed of a cemented PEEK.

Various aspects and embodiments of the present invention are defined by the following numbered clauses:
1. A glenoid implant system comprising:
   a tray component that comprises a bone-facing surface and a bearing-facing surface on opposite side of the bone-facing surface; and
   a bearing component that comprises an articulating surface that is generally concave and a back side on opposite side of the articulating surface that is generally convex,
   wherein the articulating surface is configured for engaging a convex humeral head and the back side is configured for engaging the bearing-facing surface of the tray component enabling the bearing component to slide about on the tray component while the convex humeral head articulates against the articulating surface.
2. The glenoid implant system of clause 1, wherein the tray component is formed of a metal.
3. The glenoid implant system of clause 1 or 2, wherein the bearing component is formed of a high-modulus polymer.
4. The glenoid implant system of any of the clauses 1 - 3, wherein the bearing component is formed of high-modulus polyethylene (HMPE) or ultra-high-molecular-weight polyethylene (UHMWPE).
5. The glenoid implant system of any of the clauses 1 - 4, wherein the bone-facing surface of the tray component comprises at least a portion that comprises a layer of a porous material that can promote bone in-growth.
6. The glenoid implant system of any of the clauses 1 - 5, wherein the bone-facing surface of the tray component comprises at least a portion that has a roughened surface that can accept bone cement for bonding with native bone.
7. The glenoid implant system of any of the clauses 1 - 6, wherein the bone-facing surface further comprises one or more peripheral fixation features extending therefrom in a direction that is away from the bearing-facing surface.
8. The glenoid implant system of clause 7, wherein the one or more peripheral fixation features is an annular ring.
9. The glenoid implant system of clause 8, wherein the annular ring is configured into two or more segments.
10. The glenoid implant system of clause 7 or 8, wherein the one or more peripheral fixation features are posts.
11. The glenoid implant system of any of the clauses 8 - 10, wherein the one or more peripheral fixation features further comprising one or more posts.
12. The glenoid implant system of clause 11, wherein the one or more posts extend from the annular ring.
13. The glenoid implant system of any of the clauses 7 - 12, wherein the one or more peripheral fixation features comprise a layer of a porous material that can promote bone in-growth.
14. The glenoid implant system of any of the clauses 1 - 13, wherein the back side of the bearing component is a spherical surface having a curvature defined by a first radius of curvature.
15. The glenoid implant system of clause 14, comprising a motion limiting feature that limits the range of motion of the bearing component sliding about the bearing-facing surface of the tray component.
16. The glenoid implant system of clause 15, wherein the motion limiting feature comprises the bearing-facing surface being defined by more than one radii of curvature wherein the bearing-facing surface comprises:
   a central region that is a spherical surface having a curvature defined by the first radius of curvature; and
   a peripheral portion that is a spherical surface having a curvature defined by a second radius of curvature that is smaller than the first radius of curvature.
17. The glenoid implant system of clause 15 or 16, wherein the motion limiting feature comprises a continuous ridge that extends along the bearing-facing surface's perimeter and is raised from the bearing-facing surface away from the bone-facing surface.
18. The glenoid implant system of any of the clauses 15 to 17, wherein the motion limiting feature comprises two or more ridge segments that extend along the bearing-facing surface's perimeter and are raised from the bearing-facing surface away from the bone-facing surface.
19. The glenoid implant system of any of the clauses 15 to 18, wherein the motion limiting feature comprises a boss provided on the back side of the articulating component; and a pocket provided on the bearing-facing surface of the tray component, wherein the pocket has an opening that is larger than the boss.
20. The glenoid implant system of any of the clauses 15 to 19, wherein the motion limiting
   feature comprises a pocket provided on the back side of the articulating component; and a boss provided on the bearing-facing surface of the tray component, wherein the pocket has an opening that is larger than the boss.

Also within the scope of this disclosure is a surgical kit that includes one or more of the glenoid implant systems embodiments described herein.

Although the devices, kits, systems, and methods have been described in terms of exemplary embodiments, they are not limited thereto. Rather, the appended claims should be construed broadly, to include other variants and embodiments of the devices, kits, systems, and methods, which may be made by those skilled in the art without departing from the scope and range of equivalents of the devices, kits, systems, and methods.

## Claims

1. A glenoid implant system comprising:
a tray component that comprises a bone-facing surface and a bearing-facing surface on opposite side of the bone-facing surface; and
a bearing component that comprises an articulating surface that is generally concave and a back side on opposite side of the articulating surface that is generally convex,
wherein the articulating surface is configured for engaging a convex humeral head and the back side is configured for engaging the bearing-facing surface of the tray component enabling the bearing component to slide about on the tray component while the convex humeral head articulates against the articulating surface.

2. The glenoid implant system of Claim 1, wherein the tray component is formed of a metal.

3. The glenoid implant system of Claim 1 or 2, wherein the bearing component is formed of a high-modulus polymer, and/or wherein the bearing component is formed of high-modulus polyethylene (HMPE) or ultra-high-molecular-weight polyethylene (UHMWPE).

4. The glenoid implant system of any of the Claims 1 to 3, wherein the bone-facing surface of the tray component comprises at least a portion that comprises a layer of a porous material that can promote bone in-growth, and/or wherein the bone-facing surface of the tray component comprises at least a portion that has a roughened surface that can accept bone cement for bonding with native bone.

5. The glenoid implant system of any of the Claims 1 to 4, wherein the bone-facing surface further comprises one or more peripheral fixation features extending therefrom in a direction that is away from the bearing-facing surface.

6. The glenoid implant system of Claim 5, wherein the one or more peripheral fixation features is an annular ring.

7. The glenoid implant system of Claim 6, wherein the annular ring is configured into two or more segments.

8. The glenoid implant system of Claim 5, wherein the one or more peripheral fixation features are posts.

9. The glenoid implant system of Claim 6 or 7, wherein the one or more peripheral fixation features further comprising one or more posts.

10. The glenoid implant system of Claim 9, wherein the one or more posts extend from the annular ring.

11. The glenoid implant system of any of the Claims 5 to 10, wherein the one or more peripheral fixation features comprise a layer of a porous material that can promote bone in-growth.

12. The glenoid implant system of any of the Claims 1 to 11, wherein the back side of the bearing component is a spherical surface having a curvature defined by a first radius of curvature.

13. The glenoid implant system of Claim 12, comprising a motion limiting feature that limits the range of motion of the bearing component sliding about the bearing-facing surface of the tray component.

14. The glenoid implant system of Claim 13, wherein the motion limiting feature comprises the bearing-facing surface being defined by more than one radii of curvature wherein the bearing-facing surface comprises:
a central region that is a spherical surface having a curvature defined by the first radius of curvature; and
a peripheral portion that is a spherical surface having a curvature defined by a second radius of curvature that is smaller than the first radius of curvature.

15. The glenoid implant system of Claim 13 or 14, wherein the motion limiting feature comprises at least one of:
- a continuous ridge that extends along the bearing-facing surface's perimeter and is raised from the bearing-facing surface away from the bone-facing surface;
- two or more ridge segments that extend along the bearing-facing surface's perimeter and are raised from the bearing-facing surface away from the bone-facing surface;
- a boss provided on the back side of the articulating component; and a pocket provided on the bearing-facing surface of the tray component, wherein the pocket has an opening that is larger than the boss; and
- a pocket provided on the back side of the articulating component; and a boss provided on the bearing-facing surface of the tray component, wherein the pocket has an opening that is larger than the boss.
